# FASCICULE DE BREVET EUROPEEN

(11) **EP 0 462 645 B1**
(45) Date de publication et mention de la délivrance du brevet: **17.11.1994**
(21) Numéro de dépôt: 91201421.4
(22) Date de dépôt: 10.06.1991
(51) Int. Cl.: C07C 19/08, C07C 17/00

(54) **Procédé pour la fabrication du 1,1,1,2-tétrafluoréthane**
Verfahren zur Herstellung von 1,1,1,2-tetraflourethan
Process for the preparation of 1,1,1,2-tetrafluorethane

(30) Priorité: 18.06.1990 BE 9000618
(43) Date de publication de la demande: 27.12.1991
(73) Titulaire: SOLVAY (Société Anonyme), B-1050 Bruxelles (BE)
(72) Inventeur: Janssens, Francine, B-1800 Vilvoorde (BE); Gilbeau, Patrick, B-7090 Braine-le-Comte (BE); Pennetreau, Pascal, B-1310 La Hulpe (BE)
(74) Mandataire: Meyers, Liliane

(56) Documents cités:
- EP-A- 0 353 059
- BE-A- 870 530

## Description

L'invention concerne un procédé pour la fabrication du 1,1,1,2-tétrafluoréthane (HFA-134a) par réaction du fluorure d'hydrogène avec le 1,1,2-trifluoréthylène en phase gazeuse en présence d'un catalyseur constitué d'un composé de l'aluminium.

Le brevet belge n° 870 530 divulgue un procédé pour la fabrication du 1,1,1,2-tétrafluoréthane par réaction du fluorure d'hydrogène avec le 1,1,2-trifluoréthylène en phase gazeuse en présence d'un catalyseur d'hydrofluoration constitué d'oxyde de chrome déposé sur un support ou non.

Toutefois la préparation des catalyseurs connus dans ce domaine est souvent longue, fastidieuse et difficile à réaliser industriellement. De plus ces catalyseurs perdent généralement une partie de leur activité au cours du temps, le taux de conversion diminue avec le temps de réaction.

La demande de brevet EP-0353059 décrit un procédé de préparation en phase gazeuse de 1,1-dichloro-1-fluoréthane par addition de fluorure d'hydrogène à du 1,1-dichloréthylène, en présence d'un composé d'aluminium, à une température ne dépassant pas 120 °C.

L'invention concerne un procédé mettant en oeuvre un catalyseur qui ne présente plus les inconvénients sus-mentionnés et qui conduit à une excellente sélectivité et un taux de conversion élevé. On a en effet trouvé un procédé qui présente une bonne efficacité. Dans le procédé selon l'invention peu de sous produits sont formés et la sélectivité en 1,1,1,2-tétrafluoréthane est élevée.

L'activité des catalyseurs selon l'invention est élevée et reste stable dans le temps. Les catalyseurs selon l'invention sont d'une préparation simple, ne sont pas toxiques et ont un coût très faible. Leur régénération n'est donc pas nécessaire, aucun support n'est nécessaire aux catalyseurs selon l'invention puisqu'ils jouent également le rôle de support tout en étant les catalyseurs actifs. De plus ils ne contiennent pas de métaux de transition, ce qui évite les problèmes de rejet de métaux lourds. Par ailleurs les catalyseurs sont particulièrement stables dans le temps.

L'invention concerne à cet effet un procédé pour la fabrication du 1,1,1,2-tétrafluoréthane par réaction du fluorure d'hydrogène avec le 1,1,2-trifluoréthylène en phase gazeuse en présence d'un catalyseur constitué d'au moins un composé de l'aluminium.

Dans le procédé pour la fabrication du 1,1,1,2-tétrafluoréthane selon l'invention le temps de séjour des réactifs, c'est-à-dire du fluorure d'hydrogène et du 1,1,2-trifluoréthylène, dans le réacteur est généralement d'au moins 2 secondes. Le plus souvent, ce temps de séjour est d'au moins 5 secondes. De bons résultats sont obtenus avec des temps de séjour ne dépassant pas 50 secondes. D'excellents résultats sont obtenus avec des temps de séjour ne dépassant pas 25 secondes.

La quantité de fluorure d'hydrogène mis en oeuvre dans le procédé est généralement comprise entre 0,5 et 4 moles par mole de 1,1,2-trifluoréthylène mis en oeuvre et de préférence entre 0,8 et 2,5 moles par mole de 1,1,1-trifluoréthylène mis en oeuvre.

La température à laquelle la réaction est réalisée, est supérieure à 150°C. Le plus souvent, cette température ne dépasse pas 250°C. De préférence, elle est d'au moins 200°C.

La pression à laquelle la réaction est réalisée, est choisie de manière à maintenir le milieu réactionnel à l'état de vapeur. Elle est le plus souvent voisine de la pression atmosphérique.

Le procédé de l'invention peut être réalisé dans tout réacteur ou appareillage permettant de réunir les conditions décrites ci-avant. Le procédé peut être réalisé en batch ou en continu.

Le catalyseur mis en oeuvre dans le procédé selon l'invention est constitué d'au moins un composé de l'aluminium. Généralement il est constitué par au moins 97 % d'un ou de plusieurs composés de l'aluminium, habituellement par au moins 98 % et de préférence par au moins 98,5 % d'un ou de plusieurs composés de l'aluminium. De bons résultats ont été obtenus lorsque le catalyseur est constitué exclusivement par un ou plusieurs composés de l'aluminium, c'est-à-dire qu'aucun métal de transition n'est ajouté et que les impuretés habituelles contenues dans les composés de l'aluminium ne perturbent pas le fonctionnement du catalyseur.

Généralement le catalyseur selon l'invention est constitué par un ou plusieurs sels ou oxydes d'aluminium. Habituellement il est constitué par une alumine et/ou un halogènure d'aluminium. De préférence le catalyseur est constitué par le ou les composés issus de la mise en contact d'alumine et du fluorure d'hydrogène. De bons résultats ont été obtenus lorsque, après mise en contact de l'alumine et du fluorure d'hydrogène, le catalyseur est constitué d'environ 30 à 95 % en poids de fluorure d'aluminium, de préférence d'environ 55 à 75 %.

L'invention concerne également un procédé de préparation du catalyseur. Habituellement on met en oeuvre une seule alumine mais tout type d'alumine convient. De préférence, on met en oeuvre une alumine de surface spécifique comprise entre 5 et 500 m²/g et de volume poreux compris entre 0,05 et 0,8 cm³/g. De bons résultats ont été obtenus lorsque l'on met en contact l'alumine avec le fluorure d'hydrogène jusqu'à ce qu'il n'y ait plus d'absorption de fluorure.

Le 1,1,1,2-tétrafluoréthane obtenu selon l'invention peut être utilisé dans toutes les applications connues de ce produit, telles que notamment comme réfrigérant ou propulseur d'aérosols.

Les exemples qui suivent illustrent l'invention.

### Exemple 1 :

### a) Préparation du catalyseur

On sèche de l'alumine, ayant les caractéristiques suivantes :
Alumine Harshaw (Engelhard) 0104 T 1/8" :
- surface spécifique 100 m²/g
- volume poreux 0,39 cm³/g,
pendant une nuit à 150°C sous pression réduite (étuve à vide : 5 mmHg)
On introduit 245 g de cette alumine séchée dans un réacteur, constitué d'un tube en Inconel d'une capacité de 250 cm³, chauffé au moyen de 6 résistances électriques, muni d'une gaine axiale équipée de 12 thermocouples afin de réguler et de contrôler la température tout au long du réacteur.

Puis on chauffe le réacteur à 325°C et on l'alimente à raison de 0,8 mole par heure de fluorure d'hydrogène dilué dans de l'azote (2,5 lN/h) à 325°C à pression atmosphérique.

L'alimentation en fluorure d'hydrogène est coupée lorsqu'il n'y a plus d'absorption de fluorure, c'est-à-dire lorsque le débit de fluorure d'hydrogène sortant du réacteur est égal au débit entrant.

Le catalyseur ainsi obtenu a une surface spécifique inférieure à 10 m²/g, un volume poreux de 0,05 cm³/g et un contenu en fluor d'environ 425 g/kg ce qui correspond à un degré de fluoration d'environ 50 % molaire Al (ce qui équivaut à 62 % de AlF₃ en poids).

### b) Hydrofluoration du 1,1,2-trifluoréthylène

On alimente le réacteur contenant le catalyseur préparé ci-avant à raison de 270 mmoles par heure de 1,1,2-trifluoréthylène et 520 mmoles par heure de fluorure d'hydrogène à 332°C sous pression atmosphérique. Le temps de séjour est évalué à 23 secondes.

Après 2 heures de mise en régime, le taux de conversion du 1,1,2-trifluoréthylène est de 96,4 % et la sélectivité est de 99,9 % en 1,1,1,2-tétrafluoréthane (résultats établis sur base de l'analyse par chromatographie en phase gazeuse en ligne de l'effluent gazeux sortant du réacteur).

Après 35 heures de fonctionnement, ce taux de conversion et cette sélectivité sont inchangés.

### Exemples 2, 3 et 4

### a) Préparation du catalyseur

Le catalyseur est préparée en suivant le protocole décrit à l'exemple 1a.

Le catalyseur est constitué d'alumine telle que celle décrite à l'exemple 1.

Le réacteur utilisé est identique à celui de l'exemple 1.

### b) Hydrofluoration du 1,1,2-trifluoréthylène

Les conditions de l'hydrofluoration et les résultats sont réunis dans le tableau 1.

**TABLEAU 1**

| Exemple | 2 | 3 | 4 |
|---|---|---|---|
| Température °C | 346 | 347 | 350 |
| Temps de séjour secondes | 27 | 20 | 32 |

| Résultats après 2 heures de mise en régime | | | |
|---|---|---|---|
| Taux de conversion en % du 1,1,2-trifluoréthylène | 92,5 | 89,1 | 93,7 |
| Sélectivité en % en 1,1,1,2-tétrafluoréthane | 99,95 | 99,9 | 99,95 |

Après 35 heures de fonctionnement, ce taux de conversion et cette sélectivité restent inchangés.

### Exemple 5 :

### a) Préparation du catalyseur

Dans un réacteur en Inconel de 300 cm³, placé verticalement, on introduit 252 g d'alumine Harshaw (Engelhard) 3983 T 1/8" préalablement séchée pendant 24 h à 150 °C sous pression réduite (étuve à vide : 5 mmHg).

Les caractéristiques de cette alumine sont les suivantes :
- surface spécifique 133 m²/g;
- volume poreux 0,39 cm³/g.

Le réacteur, alimenté en azote (5 lN/h), est progressivement chauffé à 200 °C, à pression atmosphérique.

On introduit ensuite, par le haut du réacteur, un courant de fluorure d'hydrogène et d'azote, à des débits valant respectivement 17,5 et 25 lN/h (rapport 0,7/1). La fluoration est poursuivie de cette manière pendant 5 heures puis, le débit d'azote est progressivement diminué à 5 lN/h et la température est portée à 350 °C.

Après un temps total de 12 heures, le sens de passage du fluorure d'hydrogène est inversé (introduction par le bas du réacteur), de manière à uniformiser le taux de fluoration. Le courant d'azote est stoppé et le débit de fluorure d'hydrogène est porté à 25 lN/h ; le traitement est poursuivi dans ces conditions pendant 11 heures.

A ce moment, il n'y a plus d'absorption de fluorure d'hydrogène, c'est-à-dire que le débit de fluorure d'hydrogène sortant du réacteur est égal au débit entrant.

A la fin du traitement, l'introduction de fluorure d'hydrogène est stoppée et le réacteur est refroidi sous balayage d'azote (5 lN/h). Le catalyseur ainsi obtenu a une surface spécifique de 15 m²/g, un volume poreux de 0,09 cm³/g et un contenu en fluor de 551 g/kg, ce qui correspond à un degré de fluoration d'environ 72 % molaire par rapport à l'aluminium (ce qui équivaut à 81 % de AlF₃ en poids).

### b) Hydrofluoration du trifluoréthylène

On alimente le réacteur rempli de catalyseur préparé ci-avant à raison de 235 mmol/h de trifluoréthylène et de 470 mmol/h de fluorure d'hydrogène, à 300 °C et à pression atmosphérique. Le temps de séjour est évalué à 10 secondes.

Après 2 heures de mise en régime, au vu de l'analyse par chromatographie en phase gazeuse en ligne de l'effluent sortant du réacteur, le taux de conversion du 1,1,2-trifluoréthylène est supérieur à 99,5 % et le 1,1,1,2-tétrafluoréthane est le seul produit détecté (sélectivité ≧ 99,9 %).

### Exemples 6 à 11

### a) Préparation du catalyseur

Le catalyseur est préparé suivant le protocole décrit à l'exemple 5a.

Le catalyseur est constitué d'alumine telle que celle décrite à l'exemple 5a.

Le réacteur utilisé est identique à celui de l'exemple 5.

### b) Hydrofluoration du trifluoréthylène

Les conditions d'hydrofluoration et les résultats sont réunis dans le tableau 2.

**TABLEAU 2**

| Exemple | 6 | 7 | 8 | 9 |
|---|---|---|---|---|
| Température °C | 250 | 200 | 250 | 250 |
| Temps de séjour secondes | 12,5 | 12,7 | 6,3 | 6,0 |
| Rapport HF/TFEe, mol/mol | 1,8 | 1,8 | 2,0 | 0,5 |

| Résultats après 2 heures de mise en régime | | | | |
|---|---|---|---|---|
| Taux de conversion en % du 1,1,2-trifluoréthylène | 99 | 73 | 91 | 50 (*) |
| Sélectivité en % en 1,1,1,2-tétrafluoréthane | ≧99,9 | ≧99,9 | ≧99,9 | ≧99,9 |

| | | | | |
|---|---|---|---|---|
| (*) Correspondant à la transformation complète du HF mis en oeuvre. | | | | |

### Exemples 10R et 11R (de référence)

### a) Préparation du catalyseur

Le catalyseur est préparé suivant le protocole décrit à l'exemple 5a.

Le catalyseur est constitué d'alumine telle que celle décrite à l'exemple 5a.

Le réacteur utilisé est identique à celui de l'exemple 5.

### b) Hydrofluoration du trifluoréthylène

Les conditions d'hydrofluoration et les résultats sont réunis dans le tableau 3.

**TABLEAU 3**

| Exemple | 10R | 11R |
|---|---|---|
| Température °C | 120 | 120 |
| Temps de séjour secondes | 16,6 | 38,8 |
| Rapport HF/TFEe, mol/mol | 2,1 | 2,5 |

| Résultats après 2 heures de mise en régime | | |
|---|---|---|
| Taux de conversion en % du 1,1,2-trifluoréthylène | ≦ 1 | ≦ 2 |
| Sélectivité en % en 1,1,1,2-tétrafluoréthane | (*) | (*) |

| | | |
|---|---|---|
| **(*)** : comme dans les exemples ci-dessus selon l'invention, le 1,1,1,2-tétrafluoréthane est le seul produit détecté ; toutefois, à si faible conversion, la teneur en 1,1,1,2-tétrafluoréthane est si faible qu'il est prudent de ne pas estimer de sélectivité. | | |

## Revendications

1. Procédé pour la fabrication du 1,1,1,2-tétrafluoréthane par réaction du fluorure d'hydrogène avec le 1,1,2-trifluoréthylène en phase gazeuse en présence d'un catalyseur caractérisé en ce que le catalyseur est constitué d'au moins un composé de l'aluminium et en ce que la température de réaction est supérieure à 150°C.

2. Procédé selon la revendication 1 caractérisé en ce que le catalyseur est constitué par au moins 97 % d'un ou de plusieurs composés de l'aluminium.

3. Procédé selon la revendication 1 ou 2 caractérisé en ce que le catalyseur est constitué par le ou les composés issus de la mise en contact d'alumine et du fluorure d'hydrogène.

4. Procédé selon la revendication 1, 2 ou 3 caractérisé en ce que le catalyseur est constitué d'environ 30 à 95 % en poids de fluorure d'aluminium.

5. Procédé selon l'une quelconque des revendications précédentes caractérisé en ce que le temps de séjour des réactifs dans le réacteur est d'au moins 2 secondes, sans dépasser 50 secondes.

6. Procédé selon l'une quelconque des revendications précédentes caractérisé en ce que la quantité de fluorure d'hydrogène mis en oeuvre est comprise entre 0,5 et 4 moles par mole de 1,1,2-trifluoréthylène.

## Claims

1. Process for the production of 1,1,1,2-tetrafluoroethane by reaction of hydrogen fluoride with 1,1,2-trifluoroethylene in the gaseous phase in the presence of a catalyst, characterised in that the catalyst is formed of at least one aluminium compound and in that the reaction temperature is above 150°C.

2. Process according to Claim 1, characterised in that the catalyst is formed of at least 97% of one or more aluminium compounds.

3. Process according to Claim 1 or 2, characterised in that the catalyst is formed of the compound or compounds resulting from contacting of alumina and hydrogen fluoride.

4. Process according to Claim 1, 2 or 3, characterised in that the catalyst is formed of approximately 30 to 95% by weight of aluminium fluoride.

5. Process according to any one of the preceding claims, characterised in that the residence time of the reagents in the reactor is at least 2 seconds, without exceeding 50 seconds.

6. Process according to any one of the preceding claims, characterised in that the quantity of hydrogen fluoride used is between 0.5 and 4 mol per mole of 1,1,2-trifluoroethylene.

## Patentansprüche

1. Verfahren zur Herstellung von 1,1,1,2-Tetrafluorethan durch Reaktion von Wasserstofffluorid mit 1,1,2-Trifluorethylen in gasförmiger Phase in Anwesenheit eines Katalyzators, dadurch gekennzeichnet, daß der Katalysator aus wenigstens einer Aluminiumverbindung gebildet ist, und daß die Reaktionstemperatur oberhalb von 150°C liegt.

2. Verfahren nach Anspruch 1, dadurch gekennzeichnet, daß der Katalysator aus wenigstens 97 % einer oder mehrerer Aluminiumverbindungen gebildet ist.

3. Verfahren nach Anspruch 1 oder 2, dadurch gekennzeichnet, daß der Katalysator aus der oder den Verbindung(en) gebildet ist, die aus dem In-Kontakt-Bringen von Aluminiumoxid und Wasserstofffluorid stammen.

4. Verfahren nach Anspruch 1, 2 oder 3, dadurch gekennzeichnet, daß der Katalysator aus etwa 30 bis 95 Gew.-% Aluminiumfluorid gebildet ist.

5. Verfahren nach einem der vorhergehenden Ansprüche, dadurch gekennzeichnet, daß die Verweildauer der Reaktionsstoffe in dem Reaktor wenigstens 2 Sekunden, jedoch nicht mehr als 50 Sekunden beträgt.

6. Verfahren nach einem der vorhergehenden Ansprüche, dadurch gekennzeichnet, daß die eingesetzte Wasserstofffluoridmenge zwischen 0,5 und 4 Mol pro Mol 1,1,2-Trifluorethylen beträgt.
